(19)

**Europäisches Patentamt**
**European Patent Office**
**Office européen des brevets**

(11) **EP 2 071 545 A1**

(12) **EUROPEAN PATENT APPLICATION**
published in accordance with Art. 153(4) EPC

(43) Date of publication:
**17.06.2009 Bulletin 2009/25**

(51) Int Cl.:
**G09B 23/28** *(2006.01)* **A61B 19/00** *(2006.01)*
**G09B 9/00** *(2006.01)*

(21) Application number: **07807151.1**

(22) Date of filing: **12.09.2007**

(86) International application number:
**PCT/JP2007/067744**

(87) International publication number:
**WO 2008/041456 (10.04.2008 Gazette 2008/15)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **29.09.2006 JP 2006266471**

(71) Applicant: **WASEDA UNIVERSITY Tokyo (JP)**

(72) Inventors:
• **TAKANISHI, Atsuo**
  **Tokyo;1698555 (JP)**
• **AIZDIN, Muhamad**
  **Tokyo;1698555 (JP)**

• **OSHIMA, Nobuki**
  **Tokyo 1698555 (JP)**
• **MIDORIKAWA, Ryu**
  **Tokyo 1698555 (JP)**
• **SOLIS, Jorge**
  **, Tokyo;1698555 (JP)**
• **OGURA, Yu**
  **Tokyo 1698555 (JP)**
• **ISHII, Hiroyuki**
  **Tokyo 1698555 (JP)**

(74) Representative: **Grünecker, Kinkeldey, Stockmair & Schwanhäusser Anwaltssozietät Leopoldstrasse 4 80802 München (DE)**

(54) **MEDICAL TECHNIQUE EVALUATION SYSTEM, TECHNIQUE EVALUATION DEVICE, TECHNIQUE EVALUATION DEVICE PROGRAM**

(57)    A tool for objectively evaluating the technique of a medical treatment such as an operation or a palpation performed by a doctor or a medical student. A medical technique evaluation system comprises a soft material member having an incision made in itself for simulative suture by a trainee such as a doctor or a medical student and elastic deformation by a force acting on the surroundings of the incision, reflective type photointerruptor for measuring the state values in three orthogonal axes according to the elastic deformation of the soft material member and a technique evaluation device for determining the evaluation score of the technique of the suture and ligation on the basis of the measured values from the reflective type photointerruptor. The technique evaluation device substitutes values based on the measured state values in the orthogonal three axes into a predetermined evaluation function to determine the evaluation score.

FIG.1

**Description**

Technical Field

The present invention relates to a medical technique evaluation system, a technique evaluation device, and a technique evaluation device program, and more particularly, to a medical technique evaluation system, a technique evaluation device, and a technique evaluation device program capable of objectively evaluating the technique of a doctor and a medical student performing various medical actions such as a treatment in surgery.

Background Art

In surgery, treatments such as incision or resection of a tissue, suture of the tissue following the incision or the resection, and suture ligation are performed. The technique related to the medical action such as these treatments requires a considerable amount of training to improve. Since the training using a human body is restricted, there has been known a surgical training simulator (see Patent Document 1) simulating a human body as a training tool for improving the surgical technique. The simulator is useful particularly for a doctor and a medical student with less experience and skill.
Patent Document 1: Japanese Patent Laid-Open No. 2005-10164

Disclosure of the Invention

Problems to be Solved by the Invention

Unfortunately, there is no tool for a less experienced doctor or medical student to objectively evaluate his or her own technique using the simulator or the like for training. Therefore, evaluation of his or her own technique requires a subjective evaluation of his or her trainer. Accordingly, in this case, technical evaluation may not be accurately performed. Further, such an evaluation always needs a trainer performing the evaluation. Thus, there is a disadvantage in that training with evaluation cannot be efficiently performed solely by himself or herself.
In view of the above disadvantage, the present invention has been devised, and an object of the present invention is to provide a doctor or a medical student with a medical technique evaluation system, a technique evaluation device, and a technique evaluation device program capable of objectively evaluating the technique of medical actions such as surgery and palpation.

Means for Solving the Problems

(1) In order to achieve the above object, the medical technique evaluation system in accordance with the present invention is configured to include: a soft material member which includes a target region on which a person to be evaluated performs a medical action simulatively and can be elastically transformed by a force acting on a periphery of the target region; a sensor which can measure state values in three orthogonal axes by elastic transformation of the soft material member; and a technique evaluation device obtaining an evaluation score of a technique for the medical action based on measured values of the sensor, wherein the technique evaluation device assigns, to the predetermined evaluation function, a value based on the individual state values in the three orthogonal axes measured by the sensor and calculates the evaluation score.

(2) Further, the configuration can be made such that the sensor is a reflective type photointerruptor having a light-emitting device and a light-receiving device; is provided in a position in which light from the light-emitting device can be reflected on the soft material member to be received by the light-receiving device; and obtains a value assigned to the evaluation function based on a change in current passing through the light-receiving device.

(3) Here, the technique evaluation device is configured to include: a voltage detecting section which detects a voltage value from the light-receiving device; a data totalization section which creates a voltage change graph showing a relation between the voltage value and the respective time from start to end of the medical action; a data computing section which obtains the assigned value from data of the data totalization section; and an evaluation score calculation section which assigns the assigned value to the evaluation function to calculate the evaluation score.

(4) Further, the evaluation function can be made into a suture technique evaluation function and/or a ligation technique evaluation function.

(5) In addition, (1) can be configured to further include a camera which grabs an image of the target region after the medical action terminates,
wherein the technique evaluation device assigns, to the predetermined evaluation function, displacement measured values based on the individual state values in the three orthogonal axes measured by the sensor and an image processing value based on image data taken by the camera to calculate the evaluation score.

(6) Here, the configuration can be made such that the sensor is a reflective type photointerruptor having a light-emitting device and a light-receiving device, and is provided in a position in which light from the light-emitting device can be reflected on the soft material member to be received by the light-receiving device,
wherein the technique evaluation device includes: a voltage detecting section which detects a voltage value from the light-receiving device; a data totalization section which creates a voltage change graph showing a relation between the voltage value and the respective time from start to end of the medical action; a data computing section which obtains the displacement measured values from data of the data totalization section; an image processing section which obtains the image processing value from image data taken by the camera; and an evaluation score calculation section which assigns the displacement measured values and the image processing value to the evaluation function to calculate the evaluation score.

(7) Further, the evaluation function can be made into a suture ligation evaluation function which comprehensively evaluates a suture technique and a ligation technique.

(8) Here, the configuration can be made such that the soft material member further includes: an artificial skin simulating a state of a human skin; and an incision formed on the artificial skin to simulate an incision portion of the skin, wherein the image processing section obtains the image processing value based on: a total open area of the incision after suture ligation treatment; spacing between individual sutures connecting an open end of the incision in multiple locations; a distance from the incision to a needle entry point in the individual suture; and a distance from the incision to a needle suture exit point in the individual suture.

(9) Further, the configuration can also be made such that the medical technique evaluation system in accordance with the present invention includes: a target region on which a person to be evaluated performs a medical action simulatively; a camera which grabs an image of the target region; and a technique evaluation device which obtains an evaluation score of a technique for the medical action based on image data taken by the camera, wherein the technique evaluation device assigns an image processing value based on the image data to the predetermined evaluation function to calculate the evaluation score.

(10) Further, the present invention is a medical technique evaluation device which obtains an evaluation score of a technique of a medical action which a person to be evaluated performs simulatively on a target region, and is configured such that the technique evaluation device obtains a value related to the technique from the individual state values in three orthogonal axes according to a transformation of the target region, assigns the value to the predetermined evaluation function, and calculates the evaluation score.

(11) Here, the configuration is preferably made to obtain a value related to the technique from image data of the target region taken by the camera following the medical action; assign, to the predetermined evaluation function, the each value related to the technique from the image data and the individual state values; and calculate the evaluation score.

(12) Further, the present invention is a program causing the medical technique evaluation device to be executed to calculate an evaluation score of a technique for a medical action which a person to be evaluated performs simulatively on a target region, and is configured to cause the technique evaluation device to perform a process of obtaining a value related to the technique from the individual state values in three orthogonal axes according to a transformation of the target region, assign the value to the predetermined evaluation function, and calculate the evaluation score.

(13) Here, the configuration is preferably made to cause the technique evaluation device to perform a process of obtaining a value related to the technique from image data of the target region taken by the camera following the medical action; assigning, to the predetermined evaluation function, the each value related to the technique from the image data and the individual state values; and calculating the evaluation score.

Advantages of the Invention

According to the present invention, a doctor or a medical student as a person to be evaluated performs a medical action such as surgery and palpation on a soft material member, and can get an evaluation score according to the capability of his or her technique, the technique of the doctor and the medical student can be quantitatively evaluated, and the training with an objective evaluation can be efficiently performed solely by himself or herself.

Brief Description of the Drawings

Figure 1 is a schematic configuration view of a medical technique evaluation system in accordance with a first embodiment;
Figure 2 is a schematic exploded plan view of a simulated body;
Figure 3 is a schematic configuration view of a medical technique evaluation system in accordance with a second embodiment;
Figure 4 is a schematic view of an image on the surroundings of an incision grabbed image after training termination; and
Figure 5 is a schematic view of the digitized image of Figure 4.

Description of Symbols

| | |
|---|---|
| 10 | Medical technique evaluation system |
| 12 | Technique evaluation device |
| 16 | Reflective type photointerruptor (sensor) |
| 18 | Soft material member |
| 20 | Light-emitting device |
| 21 | Light-receiving device |
| 24 | Artificial skin |
| 27 | Incision (target region) |
| 27A | Open end |
| 29 | Voltage detecting section |
| 31 | Data totalization section |
| 32 | Data computing section |

| 33 | Evaluation score calculation section |
| --- | --- |
| 50 | Medical technique evaluation system |
| 51 | Camera |
| 52 | Technique evaluation device |
| 55 | Image processing section |
| 56 | Data computing section |
| 57 | Evaluation score calculation section |
| 58 | Suture |
| E, $E_S$, $E_L$ | Evaluation score |
| P1 | Suture entry point |
| P2 | Suture exit point |

Best Mode for Carrying Out the Invention

Hereinafter, the embodiments of the present invention will be described with reference to the drawings.

(First embodiment)

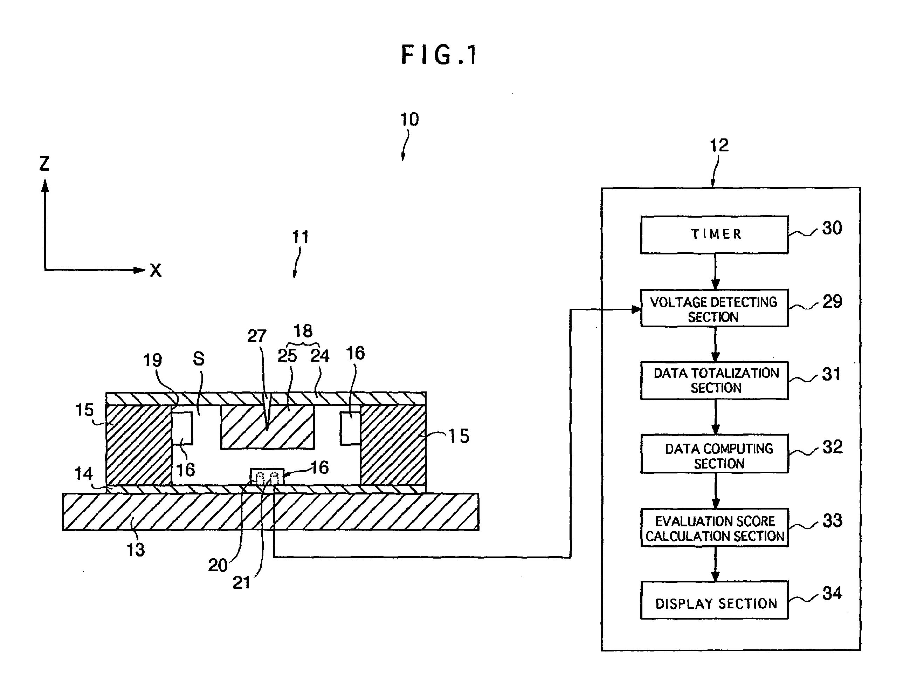

Figure 1 is a schematic configuration view of a medical technique evaluation system in accordance with a first embodiment. In this Figure, a medical technique evaluation system 10 is a system evaluating the technique of a trainee (person to be evaluated) such as a doctor and a medical student performing a training of suture treatment and ligation treatment. The medical technique evaluation system 10 includes a simulated body 11 which is used by the trainee to simulatively perform suture treatment and ligation treatment; and a technique evaluation device 12 which quantitatively evaluates the individual treatments performed on the simulated body 11.

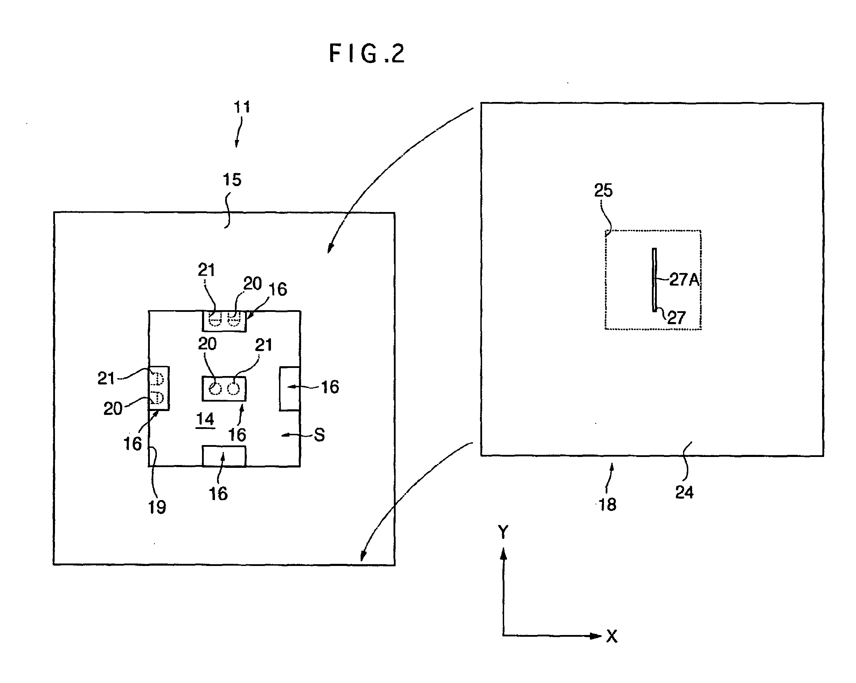

As shown in Figures 1 and 2, the simulated body 11 includes a soft material member 18 serving as the training region used for suture treatment and ligation treatment; a frame 15 supporting the soft material member 18 thereunder and having an internal space S formed in the center; a reflective type photointerruptor 16 serving as an optical sensor provided in five positions within the internal space S of the frame 15; a substrate 14 laid on the frame 15; and a base 13 on which the substrate 14 is placed.

The soft material member 18 is elastically transformable by a force acting on the individual treatment. The soft material member 18 includes an artificial skin 24 which is substantially square shaped in plan view and formed of a pudding gel simulating the state of a human skin; a urethane foam 25 which is fixed in a substantially center portion of the undersurface of the artificial skin 24 and stored in the internal space S of the frame 15; and an incision 27 which is formed in the artificial skin 24 and the urethane foam 25.

The urethane foam 25 is arranged at a predetermined spacing between the individual reflective type photointerruptors 16 in the internal space S and is formed of an elastic material difficult to penetrate light from the individual reflective type photointerruptors 16.

The incision 27 is formed so as to extend in the Y-axis direction (see Figure 2) which is in a direction orthogonal to the paper in Figure 1, and is provided so as to have a wedge shaped section deeply extending part way into the urethane foam 25 from the surface of the artificial skin 24. The incision 27 simulates an incision portion of the skin in surgery. The trainee is trained to suture and ligate the incision 27, and then the technique for the individual training is evaluated as described later. Therefore, the incision 27 constitutes a target region in which the trainee simulatively performs a treatment in surgery for a medical action.

The frame 15 has substantially the same external dimensions as the artificial skin 24, and is formed substantially square shaped in plan view. The substantially square hole shaped through hole 19 is formed near the center, and the internal side of the through hole 19 constitutes the internal space S.

The reflective type photointerruptors 16 are provided so as to surround the circumference of the urethane foam 25, one for each internal surface in the four directions on the inner circumference of the through hole 19, and one on the substrate 14 below the urethane foam 25.

These reflective type photointerruptors 16 are configured of well-known devices including a light-emitting device 20 which emits light using current from a power source (not shown); and a light-receiving device 21 which receives light emitted by the light-emitting device 20. The light-receiving device 21 is provided between a power source and ground (not shown) so as to be changed in magnitude of current passing through the light-receiving device 21 depending on the amount of light received. The upstream side (power source side) of the light-receiving device 21 has a circuit configuration in which current flows divergently into the technique evaluation device 12 side. The reflective type photointerruptor 16 includes a filter (not shown) for preventing the light-receiving device 21 from receiving external light. It should be noted that in Figures 1 and 2, the light-emitting device 20 and the light-receiving device 21 are illustrated only in part of the reflective type photointerruptors 16, and they are omitted from the rest of the reflective type photointerruptors 16.

Here, when light is emitted from the individual light-emitting device 20 of the individual reflective type photointerruptor 16, the light is reflected on the urethane foam 25 inside the reflective type photointerruptor 16 and is received at its own light-receiving device 21. When the urethane foam 25 is elastically transformed, the distance between the individual reflective type photointerruptor 16 and the urethane foam 25 is changed. Then, the amount of light received at the light-receiving device 21 is changed and the magnitude of current passing through the light-receiving device 21 is changed. Accordingly, the magnitude of current flowing to the technique evaluation device 12 side is changed. More specifically, the smaller the distance between the reflective type photointerruptor 16 and the urethane foam 25, the more increased the current passing through the light-receiving device 21, while the less the amount of current flowing to the technique evaluation device 12 side. As a result, the voltage of the circuit branching to the technique evaluation device 12 side is lowered. In other words, the smaller the degree of the transformation of the urethane foam 25, the lower the voltage of the circuit branching to the technique evaluation device 12 side.

It should be noted that, in the following description, a pair of reflective type photointerruptors 16 and 16 arranged at the left and right sides of the urethane foam 25 in Figure 2 are provided to sense the transformation of the urethane foam 25 in the X-axis direction in the same Figure orthogonal to the extending direction (Y-axis direction) of the incision 27(see Figure 1), and hereinafter referred to simply as "X-axis photointerruptor". Moreover, a pair of reflective type photointerruptors 16 and 16 arranged at the up and down sides of the urethane foam 25 in Figure 2 are provided to sense the transformation of the urethane foam 25 in the Y-axis direction along the extending direction of the incision 27, and hereinafter referred to simply as "Y-axis photointerruptor". Further, a reflective type photointerruptor 16 arranged at the bottom side of the urethane foam 25 in Figure 1 is provided to sense the transformation of the urethane foam 25 in the Z-axis direction in the same Figure along the height direction, and hereinafter referred to simply as "Z-axis photointerruptor".

The technique evaluation device 12 is configured of software and hardware including a multiple program modules and processing circuits such as a processor. The technique evaluation device 12 includes a voltage detecting section 29 which detects an individual voltage value (hereinafter referred to simply as "individual voltage value") from the light-receiving device 21 side of the individual reflective type photointerruptor 16 and converts the value to a digital signal; a timer 30 which measures time at a predetermined timing; a data totalization section 31 which creates a voltage change graph showing the relation between the voltage value and the respective time from the start of the suture treatment to the incision 27 to the end of the ligation treatment thereof; a data computing section 32 which uses the data of the data totalization section 31 to calculate a value used as an indicator of superiority of the suture technique and the ligation technique; an evaluation score calculation section 33 which uses the data of the data totalization section 31 and the data computing section 32 to calculate an individual evaluation score about the suture technique and the ligation technique; and a display section 34 which displays the calculated evaluation scores.

The voltage detecting section 29 is configured to measure the voltage value every predetermined time period (2 $\mu$sec in the present embodiment) from when the timer 30 starts measuring to when the timer 30 terminates measuring, and then, average the voltage values in units of predetermined time periods (50 msec in the present embodiment) to obtain the average voltage value.

The timer 30 operates so as to start measuring when the suture training starts, and terminate measuring when the suture treatment terminates. Then, the timer 30 operates so as to start measuring again when the ligation training starts, and terminate measuring when the ligation treatment terminates. The commands for starting and terminating are not specifically limited, but may be given manually by checking the incision 27 with eyes or may be automatically given based on the shooting of a camera (not shown). Alternatively, the timer 30 may be set to start measuring when the suture training starts, and continue measuring until the ligation treatment terminates.

The data totalization section 31 creates a voltage change graph by plotting the average voltage value calculated by the voltage detecting section 29 with respect to time. Here, the voltage value is set based on the urethane foam 25 without any transformation. When the urethane foam 25 is transformed in a direction of increasing the distance to the individual reflective type photointerruptor 16, "+" sign is assigned to the voltage value; and when the urethane foam 25 is transformed in a direction of decreasing the distance to the individual reflective type photointerruptor 16, "-" sign is assigned to the voltage value.

The data computing section 32 calculates values to be assigned to an evaluation function used by the evaluation score calculation section 33 (described later). More specifically, the values to be calculated are: a suture required time $T_S$ required from the start of a suture training to the termination thereof; a ligation required time $T_L$ required from the start of a ligation training to the termination thereof; a displacement difference $\Delta x(t)$ of the urethane foam 25 in the X-axis direction, namely, a difference between the values $X_1$ and $X_2$ detected by the X-axis reflective type photointerruptors 16 and 16; a displacement difference $\Delta y(t)$ of the urethane foam 25 in the Y-axis direction, namely, a difference between the values $Y_1$ and $Y_2$ detected by the Y-axis reflective type photointerruptors 16 and 16; a minimum value $Z_{min}$ detected by the Z-axis reflective type photointerruptor 16; a ligation count $N_L$ obtained by counting the number of peak portions on the voltage change graph at ligation operation; individual voltage values $I_x$, $I_y$, $I_z$ detected by the X-axis, Y-axis, Z-

axis reflective type photointeruptors 16 when no force is applied to the urethane foam 25; and maximum voltage values $M_x$, $M_y$, and $M_z$ detected by the X-axis, Y-axis, Z-axis reflective type photointerruptors 16 when the urethane foam 25 is transformed to the maximum, namely, the maximum portion of the peak portions at ligation operation. Here, since there are two X-axis, Y-axis photointerruptors 16 and 16 respectively, the individual voltage values $I_x$, $I_y$, $M_x$, $M_y$ are the total values of the reflective type photointerruptors 16 and 16 of the same axes.

The evaluation score calculation section 33 assigns the individual values calculated by the data computing section 32 to the predetermined evaluation function to calculate the evaluation scores of the suture technique and the ligation technique. Here, the evaluation function includes a function for suture technique evaluation and a function for ligation technique evaluation. The suture technique evaluation function is used to obtain an evaluation score $E_S$ of the suture training, and the ligation technique evaluation function is used to obtain an evaluation score $E_L$ of ligation training.

An example of the suture technique evaluation function can be given by the following expression (1).

[Expression 1]

$$E_S = \frac{\omega_1}{T_S} + \frac{\omega_2}{\left( \int_0^T |\Delta x(t)| dt \right)} + \omega_4 |Z_{min}| \qquad (1)$$

Here, the above expression (1) does not consider the displacement in the Y-axis direction, namely, the direction of extending the incision 27 since the suture operation is repeated in the X-axis direction and thus the displacement in the Y-axis direction less likely to affect the superiority of the technique than the displacement in the X-axis direction. Alternatively, an item may be added to the expression (1) to consider the displacement in the Y direction as shown in the following expression (2). In this case, the evaluation accuracy of the suture technique can be further improved.

[Expression 2]

$$E_S = \frac{\omega_1}{T_S} + \frac{\omega_2}{\left( \int_0^T |\Delta x(t)| dt \right)} + \frac{\omega_3}{\left( \int_0^T |\Delta y(t)| dt \right)} + \omega_4 |Z_{min}| \qquad (2)$$

An example of the ligation technique evaluation function can be given by the following expression (3).

[Expression 3]

$$E_L = \frac{\omega_5 N_L}{T_L} + \frac{\omega_6}{\left( \int_0^T |\Delta x(t)| dt \right)} + Max_L \qquad (3)$$

where

[Expression 4]

$$Max_L = \left| \frac{\omega_7}{|M_Z - I_Z|} \right| + \left| \frac{\omega_7}{|M_Y - I_Y|} \right| + \left| \frac{\omega_7}{|M_X - I_X|} \right| \qquad (4)$$

Here, like the expression (2), a term may be added to the expression (3) to consider the displacement in the Y direction. It should be noted that in the above individual expressions, $\omega_1$ to $\omega_7$ denote weighting coefficients, and T denotes the time of termination of the individual treatment.

Regarding these evaluation functions, for a several number of doctors and a several number of inexperienced persons, the above individual values used for variables are obtained in advance and a well-known discriminant analysis is performed. Then, the well-known discriminant analysis is used to calculate the individual coefficients $\omega_1$ to $\omega_7$ so as to be judged that the greater the evaluation scores $E_S$ and $E_L$, the better the suture technique and the ligation technique. It should be noted that any part of or all of the coefficients $\omega_1$ to $\omega_7$ may be arbitrarily set.

It should be noted that as a variable of the evaluation function, any variable can be used regardless of the type and number thereof as long as it can affect the superiority of the suture technique and the ligation technique. In that case, an evaluation function is obtained separately using a discriminant analysis and the evaluation function may be applied.

Therefore, according to such a first embodiment, when training is performed to suture and ligate the incision 27 of the soft material member 18, the technique evaluation device 12 having a relatively simple configuration can be used to automatically obtain the evaluation scores $E_S$ and $E_L$ indicating the suture technique and the ligation technique, and thus an objective evaluation of the suture technique can be easily obtained without accompanying a trainer.

Hereinafter, another embodiment of the present invention will be described. It should be noted that in the following description, the same or identical elements as those of the first embodiment are designated with the same reference characters, and the description is omitted or simplified.

(Second embodiment)

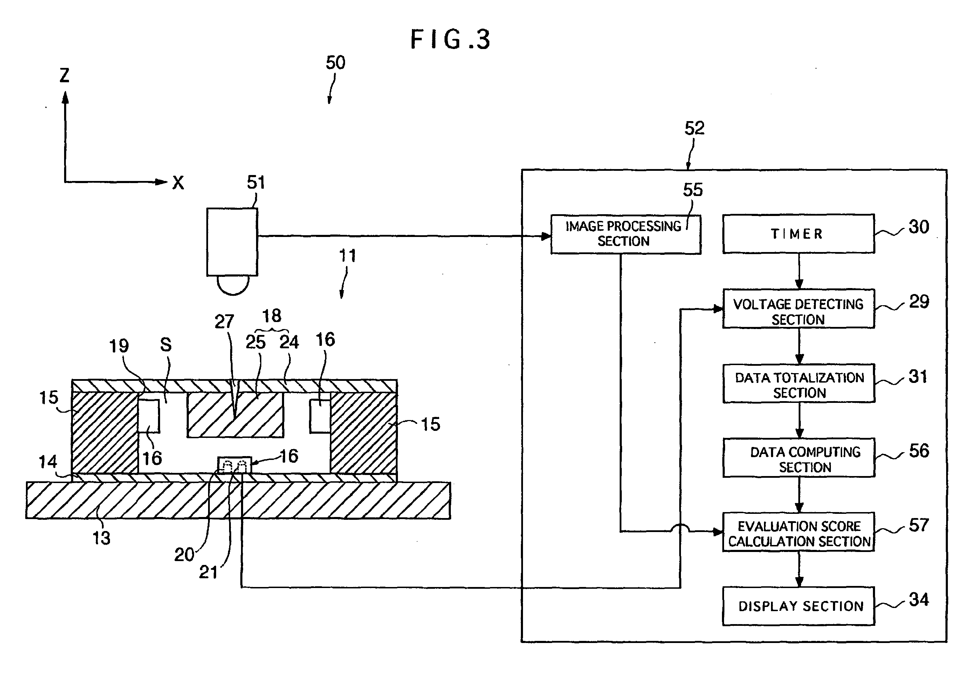

The medical technique evaluation system 50 in accordance with the second embodiment shown in Figure 3 uses an evaluation function different from that of the system 10 in accordance with the first embodiment to obtain a comprehensive evaluation score E of the suture technique and the ligation technique.

More specifically, the present embodiment uses the suture state of a treatment region following the suture treatment and the ligation treatment as an evaluation element of the suture technique and the ligation technique. That is, the present embodiment is characterized to newly add, as the evaluation element, an element about a portion (open portion) of the incision 27, the surface of which remains open without being sutured viewed from the surface side following individual treatments; and an element about suture arrangement state after ligation. The reason for adding the elements is that it is not good to have an open portion where the skin is sutured after suture treatment and ligation treatment; it is good to have the individual suture spaced at constant pitch (evenly) in a several number of skin portions crossing the incision portion; and further, it is good to have balanced suture such that the suture entry point and the suture exit point at both ends of the individual suture are arranged symmetrically with a junction of the incision portion therebetween.

In addition to the system 10 in accordance with the first embodiment, the medical technique evaluation system 50 further includes a camera 51 capable of grabbing an image of the incision 27 following suture treatment and ligation treatment, and replaces the technique evaluation device 12 of the first embodiment with a technique evaluation device 52 having partially different configuration. The rest of the configuration is substantially the same as the system 10.

The camera 51 is arranged so as to be able to grab an image of the surface of the incision 27 from above the incision 27.

In addition to the first embodiment, the technique evaluation device 52 further includes an image processing section 55 for processing image data taken by the camera 51, and replaces the data computing section 32 and the evaluation score calculation section 33 with a data computing section 56 and an evaluation score calculation section 57 improving the processing of the data computing section 32 and the evaluation score calculation section 33. The rest of the configuration is substantially the same as the technique evaluation device 12.

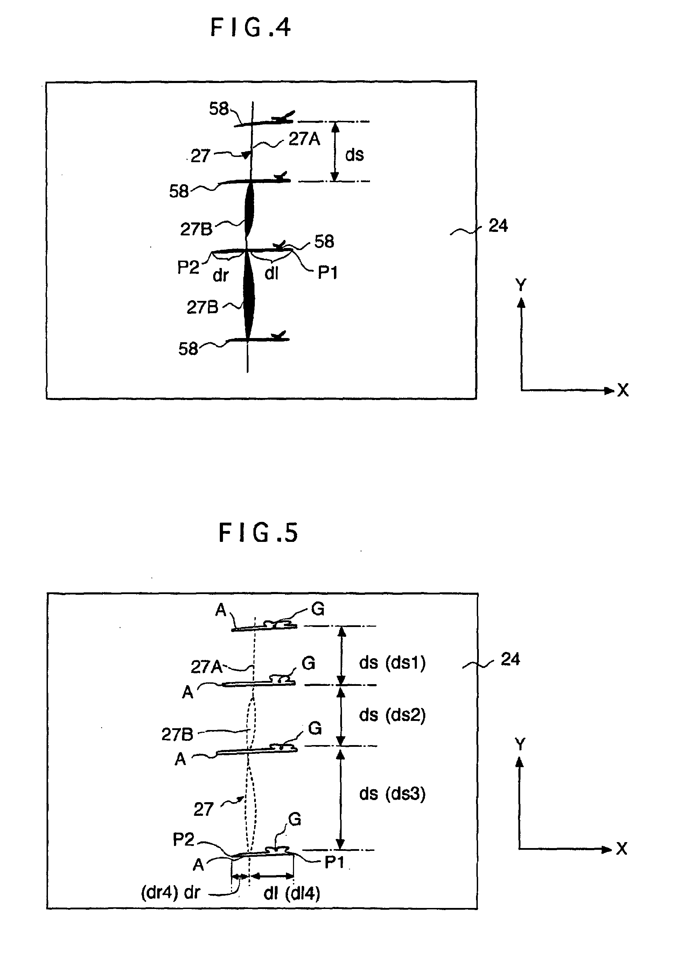

The image processing section 55 uses the image data taken by the camera 51 following suture treatment and ligation treatment to obtain the following image processing values. As shown in Figure 4, the image processing values include: a total open area S of the open portions 27B where the left and right open ends 27A in the same Figure of the open end 27A of the incision 27 remain open without being connected; a spacing ds of the sutures 58 used to suture a several number of portions crossing the incision 27; a distance dl (hereinafter referred to as "suture entry side distance dl") between the incision 27 and a needle entry point P1 regarding the individual suture 58; and a distance dr (hereinafter referred to as "suture exit side distance dr") between the incision 27 and a needle exit point P2. Here, for the following image processing, the artificial skin 24, the open end 27A of the incision 27 and the inner portion thereof, and suture 58 are differently colored from each other. The color may be selected differently as long as the following processing can be performed, but according to the present embodiment, the artificial skin 24 is white, the open end 27A of the incision 27 and the inner portion thereof are red, and suture 58 is blue.

The total open area S is obtained as follows. First, a well-known image processing method is used to convert the image data to HSV color space, and then, a red portion is extracted. Here, red is a color assigned to the open end 27A of the incision 27 and the inner portion thereof. Thus, the region in which the open portion 27B is present is expressed by being painted in red on the image, and the region where the left and right open ends 27A are connected is expressed by a red line on the image. In other words, the extracted red portions are the regions of the junction portion of the open end 27A of the incision 27 and the open portion 27B. Next, digitization is performed to convert the extracted red portions to white and convert the rest of the portions to black. Then, the total number of pixels of the red portions converted to white is counted to obtain the total open area S. At this time, the coordinate on the image of the incision 27 becoming the red portion is calculated.

The spacing ds of the sutures 58 is obtained as follows. First, as in the same manner as the total open area S is obtained, the image converted to HSV color space is used to extract a blue portion indicating the suture 58. Next, digitization is performed to convert the blue portion to white and convert the rest of the portions to black. It should be understood that Figure 5 is a schematic view of the digitized image, but in the Figure, the black portion is expressed in white. Then, the digitized image is scanned from above the image to store the coordinate on the image of a white portion corresponding to the suture 58. In the process of scanning, successive white portions are recognized as a presence region A of one suture 58, and then, the number of presence regions A, namely, the number of sutures is obtained. Then, the coordinate of the center of gravity of the white portion is obtained for each presence region A, and the coordinate is determined as the center of gravity position G of the suture 58 for each presence region A. Then, the coordinates of the center of gravity position G of the individual suture 58 are used to compute the spacing between the individual center of gravity positions G, and the spacing is determined as the spacing ds of the individual suture 58.

The digitized image used to obtain the spacing ds of the suture 58 and the above described coordinate of the open end 27A of the incision 27 are used to obtain the suture entry side distance dl and the suture exit side distance dr for each presence region A as follows. Here, the right side end in Figure 5 of the white portion constituting the individual presence region A is assumed to be the suture entry point P1; the left side end thereof is assumed to be the suture exit point P2; the left and right direction in Figure 5 is assumed to be the X coordinate on the image; and the up and down direction in the same Figure is assumed to be the Y coordinate on the image. Then, the minimum spacing in the X coordinate between the suture entry point P1 and the point at which the Y coordinate of the open end 27A of the incision 27 matches the suture entry point P1 is obtained and the spacing is determined to be the suture entry side distance dl. Then, the minimum spacing in the X coordinate between the suture exit point P2 and the point at which the Y coordinate of the open end 27A of the incision 27 matches the suture exit point P2 is obtained and the spacing is determined to be the suture exit side distance dr.

The data computing section 56 obtains a total required time T from when the suture treatment starts to when ligation treatment terminates; and displacement measured values based on the individual state values in the three orthogonal axes measured by the reflective type photointerruptor 16. The displacement measured values include: the displacement difference $\Delta x(t)$ which is a difference between the individual detected values $X_1$ and $X_2$ detected by the X-axis reflective type photointerruptors 16 and 16; the displacement difference $\Delta y(t)$ which is a difference between the individual detected values $Y_1$ and $Y_2$ detected by the Y-axis reflective type photointerruptors 16 and 16; a minimum value $Z_{min}$ of the detected values detected by the Z-axis reflective type photointerruptor 16; and the individual voltage values $X_1(T)$, $X_2(T)$, $Y_1(T)$, $Y_2(T)$, and $Z(T)$ detected by the X-axis, Y-axis, and Z-axis reflective type photointerruptors 16 respectively when the ligation treatment is terminated, namely, the training is terminated.

The evaluation score calculation section 57 assigns the image processing value obtained by the image processing section 55, and the total required time T obtained and the displacement measured values by the data computing section 56 to a suture ligation evaluation function to evaluate the suture technique and the ligation technique in a comprehensive manner, to obtain the evaluation score E. It is comprehensively judged that the greater the evaluation score E, the better the suture technique and the ligation technique.

An example of the suture ligation technique evaluation function can be given by the following expression (5). In the above expression (5), $\omega_1$ to $\omega_7$ denote weighting coefficients, and the individual coefficients $\omega_1$ to $\omega_7$ are obtained in the same manner as in the first embodiment. It should be noted that any part of or all of the coefficients $\omega_1$ to $\omega_7$ may be arbitrarily set.

[Expression 5]

$$E = \frac{\omega_1}{T} + \frac{\omega_2}{\left( \int_0^T \left( |\Delta x(t)| + |\Delta y(t)| \right) dt \right)} + \omega_3 \left| Z_{min} \right|$$

$$+ \frac{\omega_4}{|X_1(T)| + |X_2(T)| + |Y_1(T)| + |Y_2(T)| + |Z(T)|}$$

$$+ \frac{\omega_5}{\sigma^2} + \frac{\omega_6}{\sum_{i=1}^{N} |dl_i - dr_i|} + \frac{\omega_7}{S} \qquad (5)$$

Here, σ denotes a standard deviation indicating a variation of the spacing ds of the individual sutures 58, and can be calculated by the following expressions (6) and (7). It should be noted that the subscript "i" used in the expressions (5) to (7) corresponds to a presence region A in the order i=1, 2... N, starting with the presence region A at the top of the images to downward. The character "n" in the expressions (6) and (7) denotes the total number of presence regions A, namely, the number of sutures.

[Expression 6]

$$\overline{ds} = \frac{1}{n} \sum_{i=1}^{N} ds_i \qquad (6)$$

$$\sigma^2 = \frac{1}{n} \sum_{i=1}^{N} (ds_i - \overline{ds})^2 \qquad (7)$$

According to the medical technique evaluation system 50 of the above second embodiment, as an evaluation element of the suture technique and the ligation technique, the first term of the expression (5) considers the total treatment time; the second term considers the amount of displacement of the target portion in a plane direction at suture treatment; the third term considers whether or not the suture is inserted into a deep position at suture treatment; the fourth term considers whether the transformation of the suture portion after ligation treatment is large or small; the fifth term considers the variation of suture spacing after ligation treatment; the sixth term considers the left and right balancing between the suture entry point P1 and the suture exit point P2 after ligation treatment; and the seventh term considers the presence or absence of the open portion 27B after ligation treatment. Therefore, more evaluation elements than those in the first embodiment are considered and thus more accurate evaluation score E of the suture technique and the ligation technique can be obtained.

It should be noted that according to the second embodiment, the image processing value obtained by the image processing section 55 and the displacement measured values obtained by the data computing section 56 are used to obtain the evaluation score E, but only the image processing value may be used to obtain the evaluation score E. In this case, an example of the evaluation function can be given by the expression (5) from which the second to fourth terms are deleted.

According to the above individual embodiments, the reflective type photointerruptor 16 is used as a sensor for measuring the transformation of the soft material member 18, but the present invention is not limited to this, and any sensor may be used as long as the sensor can measure a predetermined state quantity corresponding to the transformation of the soft material member 18. For example, an ultrasonic sensor or the like may be applied instead. Alternatively, a plurality of reflective type photointerruptors 16 may be provided to obtain further accurate technique evaluation.

Further, the present invention allows the shape of the soft material member 18 to be adjusted so as to evaluate another technique for other medical actions such as a technique for other surgeries such as amputation or palpation or the like.

Further, the soft material member 18 is not limited to the configuration of the above individual embodiments, but any material member may be used as long as the material member is elastically transformable according to the technique to be evaluated. However, if the reflective type photointerruptor 16 is used like the above embodiments, it is preferably formed of a material excellent in light blocking effect.

Still further, the configuration of the individual sections of the devices in accordance with the present invention is not limited to the illustrated configuration, but various modifications may be made as long as substantially identical effect can be exerted.

## Claims

1. A medical technique evaluation system comprising: a soft material member which includes a target region on which a person to be evaluated performs a medical action simulatively and can be elastically transformed by a force acting on a periphery of the target region; a sensor which can measure state values in three orthogonal axes by elastic transformation of the soft material member; and a technique evaluation device which obtains an evaluation score of a technique for the medical action based on measured values of the sensor,
   wherein the technique evaluation device assigns, to the predetermined evaluation function, a value based on the individual state values in the three orthogonal axes measured by the sensor and calculates the evaluation score.

2. The medical technique evaluation system according to claim 1, the sensor is a reflective type photointerruptor having a light-emitting device and a light-receiving device; is provided in a position in which light from the light-emitting device can be reflected on the soft material member to be received by the light-receiving device; and obtains a value assigned to the evaluation function based on a change in current passing through the light-receiving device.

3. The medical technique evaluation system according to claim 2, the technique evaluation device comprises: a voltage detecting section which detects a voltage value from the light-receiving device; a data totalization section which creates a voltage change graph showing a relation between the voltage value and the respective time from start to end of the medical action; a data computing section which obtains the assigned value from data of the data totalization section; and an evaluation score calculation section which assigns the assigned value to the evaluation function to calculate the evaluation score.

4. The medical technique evaluation system according to claim 1, 2, or 3, the evaluation function is a suture technique evaluation function and/or a ligation technique evaluation function.

5. The medical technique evaluation system according to claim 1, further comprising a camera which grabs an image from the target region after the medical action terminates,
   wherein the technique evaluation device assigns, to the predetermined evaluation function, displacement measured values based on the individual state values in the three orthogonal axes measured by the sensor and an image processing value based on image data taken by the camera to calculate the evaluation score.

6. The medical technique evaluation system according to claim 5, the sensor is a reflective type photointerruptor having a light-emitting device and a light-receiving device, and is provided in a position in which light from the light-emitting device can be reflected on the soft material member to be received by the light-receiving device,
   wherein the technique evaluation device comprises: a voltage detecting section which detects a voltage value from the light-receiving device; a data totalization section which creates a voltage change graph showing a relation between the voltage value and the respective time from start to end of the medical action; a data computing section which obtains the displacement measured values from data of the data totalization section; an image processing section which obtains the image processing value from image data taken by the camera; and an evaluation score calculation section which assigns the displacement measured values and the image processing value to the evaluation function to calculate the evaluation score.

7. The medical technique evaluation system according to claim 6, the evaluation function is a suture ligation evaluation function which comprehensively evaluates a suture technique and a ligation technique.

8. The medical technique evaluation system according to claim 7, the soft material member further comprises: an artificial skin simulating a state of a human skin; and a incision formed on the artificial skin to simulate an incision portion of the skin,

wherein the image processing section obtains the image processing value based on: a total open area of the incision after suture ligation treatment; spacing between individual sutures connecting an open end of the incision in multiple locations; a distance from the incision to a needle entry point in the individual suture; and a distance from the incision to a needle suture exit point in the individual suture.

9. A medical technique evaluation system comprising: a target region on which a person to be evaluated performs a medical action simultaneously; a camera which grabs an image of the target region; and a technique evaluation device which obtains an evaluation score of a technique for the medical action based on the image data taken by the camera, wherein the technique evaluation device assigns an image processing value based on the image data to the predetermined evaluation function to calculate the evaluation score.

10. A medical technique evaluation device which obtains an evaluation score of a technique of a medical action which a person to be evaluated performs simultaneously on a target region, the technique evaluation device obtains a value related to the technique from individual state values in three orthogonal axes according to a transformation of the target region, assigns the value to the predetermined evaluation function, and calculates the evaluation score.

11. The technique evaluation device according to claim 10, obtaining a value related to the technique from image data of the target region taken by the camera following the medical action; assigning, to the predetermined evaluation function, the each value related to the technique from the image data and the individual state values; and calculating the evaluation score.

12. A technique evaluation device program to be executed on a medical technique evaluation device which obtains an evaluation score of a technique for a medical action which a person to be evaluated performs simultaneously on a target region, causing the technique evaluation device to perform a process of obtaining a value related to the technique from the individual state values in three orthogonal axes according to a transformation of the target region, assigning the value to the predetermined evaluation function, and calculating the evaluation score.

13. The technique evaluation device program according to claim 12, causing the technique evaluation device to perform a process of obtaining a value related to the technique from image data of the target region taken by the camera following the medical action; assigning, to the predetermined evaluation function, the each value related to the technique from the image data and the individual state values; and calculating the evaluation score.

# FIG.1

EP 2 071 545 A1

FIG.2

EP 2 071 545 A1

FIG.3

50

51

11

X

Z

S

15 16 18 24 25 27 19 16 16 20 21 13 14 15

52

55 IMAGE PROCESSING SECTION

30 TIMER

29 VOLTAGE DETECTING SECTION

31 DATA TOTALIZATION SECTION

56 DATA COMPUTING SECTION

57 EVALUATION SCORE CALCULATION SECTION

34 DISPLAY SECTION

## F I G . 4

## F I G . 5

**INTERNATIONAL SEARCH REPORT**

| International application No. |
| --- |
| PCT/JP2007/067744 |

A. CLASSIFICATION OF SUBJECT MATTER
*G09B23/28*(2006.01)i, *A61B19/00*(2006.01)i, *G09B9/00*(2006.01)i

According to International Patent Classification (IPC) or to both national classification and IPC

B. FIELDS SEARCHED

Minimum documentation searched (classification system followed by classification symbols)
G09B23/28-23/34, A61B19/00, G09B9/00

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2004-348095 A (National Institute of Advanced Industrial Science and Technology), 09 December, 2004 (09.12.04), Full text; Figs. 1 to 8 & WO 2004/086335 A1 | 9-13<br>1,4-5 |
| Y | JP 2005-121889 A (Kokuritsu Daigaku Hojin Gifu Daigaku), 12 May, 2005 (12.05.05), Abstract (Family: none) | 1,4-5 |
| A | JP 2000-132089 A (Kabushiki Kaisha Morita Seisakusho), 12 May, 2000 (12.05.00), Claim 1; Par. Nos. [0034] to [0037] (Family: none) | 1-13 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>    01 October, 2007 (01.10.07) | Date of mailing of the international search report<br>    09 October, 2007 (09.10.07) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**EP 2 071 545 A1**

| | INTERNATIONAL SEARCH REPORT | International application No. |
|---|---|---|
| | | PCT/JP2007/067744 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2005-525598 A  (Haptica Ltd.),<br>25 August, 2005 (25.08.05),<br>Full text; Figs. 1 to 10<br>& WO 2003/096307 A1     & US 2005/0084833 A1<br>& EP 1504431 A0          & CA 2484586 A1 | 1-13 |

Form PCT/ISA/210 (continuation of second sheet) (April 2007)

18

**EP 2 071 545 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005010164 A